# EUROPEAN PATENT SPECIFICATION

(11) **EP 3 700 352 B1**
(45) Date of publication and mention of the grant of the patent: **22.09.2021**
(21) Application number: 17788203.2
(22) Date of filing: 23.10.2017
(51) Int. Cl.: A23L 2/54, A23L 2/60, A23G 3/36, A61K 9/48, A23L 27/60, A23L 27/30

(54) **AROMA COMPOSITION**
AROMAZUSAMMENSETZUNG
COMPOSITION D'ARÔME

(43) Date of publication of application: 02.09.2020
(73) Proprietor: Symrise AG, 37603 Holzminden (DE)
(72) Inventor: HENTSCHEL, Fabia, 37603 Holzminden (DE); LEY, Jakob, 37603 Holzminden (DE); RIESS, Thomas, 37603 Holzminden (DE); PAETZ, Susanne, 37671 Höxter (DE); FAHLE, Jens, 37627 Heinade (DE); OBST, Katja, 04229 Leipzig (DE)
(74) Representative: Eisenführ Speiser
(86) International application number: PCT/EP2017/076975
(87) International publication number: WO 2019/080990

(56) References cited:
- US-A- 3 743 716
- US-A- 3 751 270
- US-B2- 8 992 892
- DUBOIS G E ET AL: "Dihydrochalcones Sweeteners. Synthesis and Sensory Evaluation of Sulfonate Derivatives", JOURNAL OF AGRICULTURAL AND FOOD CHEMI, AMERICAN CHEMICAL SOCIETY, BOOKS AND JOURNALS DIVISION, vol. 25, no. 4, 1 January 1977 (1977-01-01), pages 763-772, XP002482285, ISSN: 0021-8561

## Description

### FIELD OF INVENTION

The present invention relates to the area of oral compositions, in particular beverages and provides new aroma compositions for preparing preparations with high sweetness and low caloric intake.

### STATE OF THE ART

Foodstuffs or semi-luxury products, with a high sugar content (especially sucrose (=saccharose), lactose, glucose or fructose or mixtures of these), are generally particularly preferred by consumers because of their sweetness. On the other hand, it is well known that a high content of carbohydrates which are easy to metabolize results in a sharp increase in the blood sugar level, and the formation of fatty deposits and ultimately can lead to health problems such as obesity, adiposity, insulin-resistance, adult-onset diabetes and the associated conditions of these. In particular this is compounded by the fact that many of the stated carbohydrates can also have an adverse effect on dental health, since they are broken down in the oral cavity by certain kinds of bacteria into lactic acid, for example, which can attack the dental enamel of young and adult teeth (tooth decay).

It has therefore long been an objective to reduce the sugar content of foodstuffs and/or semi-luxury foods to the absolute minimum or below. One such measure is the use of sweeteners. These are substances which in themselves have no or only a very low calorific value and which at the same time bring about a strong sweet taste impression; these substances are as a rule non-cariogenic (an overview can be found, by way of example, in the Journal of the American Dietetic Association 2004, 104 (2), 255-275).

So-called bulk sweeteners such as sorbitol, mannitol or other sugar alcohols are indeed to some extent exceptional sweeteners and can also partly replace the other characteristics of sugar for food technology purposes, but if these are ingested too often they can cause osmotic related digestive problems in some people.

Non-nutritious, highly intensive sweeteners are in fact, due to their low usage concentration, well suited to providing sweetness in foodstuffs, but often demonstrate taste problems due to a time-intensity profile that differs from that of sugar (e.g. sucralose, steviosides, cyclamate), a bitter and/or astringent aftertaste (e.g. acesulfame K, saccharin, stevioside, rebaudioside) and/or pronounced additional flavor impressions (e.g. glycyrrhizic acid ammonium salt). Some of these sweeteners are not particularly stable under heat (e.g. thaumatin, brazzein, monellin), are not stable in all applications (e.g. aspartame) and in some cases have a long-lasting sweetening effect (strong sweet aftertaste, e.g. saccharin, sucralose). An alternative--without using non-nutrient sweeteners--consists of lowering the sugar content of foodstuffs and/or semi-luxury foods and using sensorically weak or imperceptible substances, which intensify the sweetness indirectly or directly.

In WO 2007/014879 A1 the use of hesperetin and in WO 2007/107596 A1 phloretin as an intensifier of the sweet flavor of sugar-reduced preparations for food or pleasure is recommended. Occasionally, however, when using hesperetin and phloretin the comparative weakness of the intensification of sweetness in foodstuffs and semi-luxury foods e.g. in yogurt products, containing high proportions of proteins, in particular denatured proteins or polysaccharides, can be a disadvantage. Hesperetin also has the disadvantage in very acidic and carbonized applications such as lemonades and cola drinks, that it is not sufficiently effective. Regarding hesperetin, additional reference is made to patent applications US 3 751 270 A and US 3 743 716 A, pertaining to sweetening compositions comprising hesperetin dihydrochalcone in combination with other ingredients.

Further, foodstuffs or semi-luxuries often contain various unpleasant-tasting substances, e.g. bitter substances, strongly sour substances and astringent substances, which on the one hand in moderation are desirable and characteristic (e.g. caffeine in tea or coffee, tannins in red wine or green tea, quinine in so-called bitter-lemon beverages, saponins or isoflavonoids or glycosides thereof in soya milk, hop extracts in beer, fruit acids or edible acids in sweet fruit juices), but on the other hand can also greatly reduce the value (e.g. flavonoid glycosides and limonoids in citrus juices, bitter and/or astringent aftertaste of many artificial sweeteners such as aspartame or saccharin, hydrophobic amino acids and/or peptides in cheese, fruit acids or edible acids without sufficient toning down by sweet flavoring materials, e.g. in milk products containing lactic acid). Often the unpleasant taste is further intensified by unpleasant odors, for example in soya milk, which often has a bitter and astringent taste, a note generally designated as "beany" is also described as unpleasant.

Bittertaste is regularly caused by particular substances, which bind to special bitter receptors on taste cells (which are to be found in the so-called taste buds on the tongue) and, via neurochemical cascades, send a signal to the brain, which causes a defense reaction and a negative taste impression (cf. Wolfgang Meyerhof, Reviews of Physiology, Biochemistry and Pharmacology 2005, 154, 37-72).

Astringent taste is as a rule caused by precipitation of proline-rich proteins in the saliva by astringents, e.g. metal salts or tannins. The normally homogeneous saliva that serves as a "lubricant" then contains denatured proteins, which reduce the lubricity and so leave a rough or dry sensation in the mouth, which is also experienced as astringent (Isabelle Lesschaeve, Ann C. Noble, American Journal of Clinical Nutrition 2005, 81, 330S-335S) .

Sour taste is caused by protic acids. The so-called titratable proton concentration is then more decisive than the pH for the sour impression: for example, a hydrochloric acid solution with the same pH as a malic acid solution tastes far less sour in comparison. Classically, the aversive sour taste is toned down considerably by combining with sweet flavoring materials, principally sugar, or even by substances that taste salty, mainly sodium chloride, whereas the sour taste is perceived as much more unpleasant with bitter or astringent tasting substances. However, sweet-tasting substances (for example sweeteners) are regularly used at comparatively high concentrations, thus as a rule in an amount which, with respect to their sweet taste impression, would correspond to an at least 2 wt.-percent aqueous sucrose solution, to achieve a marked toning-down of the sour impression.

Some fruit acids, in particular citric acid, succinic acid, malic acid and tartaric acid, also produce a sensory impression described as astringent, along with the sour taste.

In some foodstuffs, in particular foodstuffs derived from sweet-sour fruits or vegetables (e.g. fruit juices, fruit preparations and foodstuffs produced from them) and products produced by fermentation by acid-producing microorganisms (e.g. yogurt, ghee, kefir, soya-yogurt, sauerkraut, sourdough bread, sausages, sour milk, cheese, mixed pickles, refreshing drinks containing lactobionic acid or glucuronic acid), the acid content is necessary to produce microbial stability. Up to a certain degree this is accepted as regards taste, but in many cases there is a desire to achieve a reduced sour sensory impression without affecting the pH, which is required for the keeping qualities. We must also consider foodstuffs for which, in order to achieve a sufficient microbial or also antioxidative stability, the pH is adjusted with fruit acids or edible acids (e.g. apple juice with ascorbic acid, refreshing drinks with citric or phosphoric acid, dressings and ketchup with acetic acid), but the sour taste is regularly perceived as too strong and should therefore be reduced in sensory terms.

To summarize, it can be stated that there are various foods or foodstuffs that produce a sour sensory impression that is too high, i.e. higher than desired, which is caused by the natural concentration of fruit acids, acids formed by fermentation or acids added for reasons of stability, and where the pH of the foods or foodstuffs cannot or should not be altered for technological reasons (microbial stability, antioxidative stability etc., as explained above).

It has been described in the prior art that certain proteins such as miraculin can, in the presence of acids, transform the sour impression more or less into a sweet taste impression (http://de.wikipedia.org/wiki/Miraculin). However, because of conversion to the sweet taste, which is undesirable in non-sweet applications, this solution is only of limited use and moreover lasts too long, at 2-4 h, so that this effect is only of very restricted benefit.

Non-nutrient, highly intensive sweeteners often exhibit taste problems. The steviol glycosides (for example stevioside, rebaudioside A-Z [A, B, C, D, E, F, G, H, I, O, M, N, V, W, X, Z, KA, etc.], steviolbioside, dulcoside A, dulcoside B, rubusoside, suavioside A, B and G-J) naturally occurring in *Stevia* ssp. or *Rubus* ssp., while being very good sweeteners, at the concentrations necessary for an adequate sweetening effect (for example 400-600 ppm for rebaudioside A [purity>90%] in soft drinks, in order to achieve a sweetness corresponding to a concentration of sucrose of 10% by weight) already exhibit a pronounced licorice-like and unpleasant bitter and astringent off-taste and/or aftertaste.

In particular in sweet, calorie-free or low-calorie drinks, which have been manufactured with the help of such sweeteners, this unpleasant off-taste and/or aftertaste frequently lowers the sensory acceptance and should therefore be masked.

In the literature a number of possibilities have been offered for this. Thus in US 2004 0142084 alkaline metal hydrogen sulfates are described as masking agents. These increase the acid content in applications sharply, however. In US 3,924,017 caffeic acid derivatives have been proposed for masking. The disadvantage is that caffeic acid itself has a slightly bitter taste and easily suppresses the sweetness, so that more sweeteners would have to be used.

In WO 2006/087991 the unpleasant taste is suppressed by alkamides such as spilanthol; often, however, the tingling effect of this substance group is not desired here so that these do not have wide application.

An improvement in the taste features, in particular concerning the problem of aftertaste of non-nutrient, high intensity sweeteners can be achieved by using tannic acid, e.g. as described in WO 1998 020753 A1**,** or phenolic acids, e.g. as described in US 3,924,017**.** However, because of their catechol units such substances are not particularly stable in applications and as typical astringents intensify a bitter and/or astringent off-taste and/or aftertaste.

Not only the above-mentioned steviol glycosides, but also other substances, with a bitter taste or aftertaste, can in foodstuffs or semi-luxury foods sharply reduce the quality of these (e.g. flavonoid glycosides and limonoids in citrus juices, artificial sweeteners such as aspartame or saccharin, hydrophobic amino acid and/or peptides in cheese), even though substances with such taste directions may be desirable in moderation and characteristic of such foodstuffs or semi-luxury foods (e.g. caffeine in tea and coffee, quinine in so-called bitter lemon drinks, hop extracts in beer).

In particular to lower the natural content of bitter substances a subsequent treatment is therefore often necessary, for example extractively such as with the decaffeination of tea or coffee, or enzymatically, such as for example with the treatment of orange juice with a glycosidase in order to destroy the bitter naringin or use of special peptidases in the ripening of cheese. This treatment places a strain on the product, generates waste materials and also causes, for example, solvent residues and other residues (enzymes) in the products.

The relationships between structure and sweetening power were investigated as far back as 1979 *(*J. Chem. Senses 1979, 4(1), 35-47**).** It was found that the 3-hydroxy-4-methoxy-phenyl group represents an important condition for a powerful sweetener, and reversing the substituents is associated with a loss of sweetening power. The intensively sweet tasting dihydrochalcone (2) and the surprisingly tasteless dihydrochalcone (3) are presented in this publication. Hesperetin dihydrochalcone (I) itself, as well as potential masking or sweetness intensifying features of these compounds are not described.

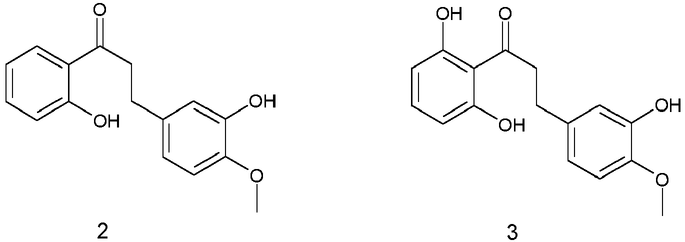

The compound (I) itself is known from the literature and is described, inter alia, in J. Agric. Food Chem. 1977, 25(4), 763-772**,** as a sweet-tasting substance. This publication is however focused on sulfonate derivatives of hesperetin dihydrochalcone (I) and their sensory evaluation. No further sensory effects of compound (I) are described.

Compound (I) is likewise mentioned in the publication J. Med. Chem. 1981, 24(4), 408-428**,** which similarly deals with sweeteners based on a dihydrochalcone structure. The importance of the 3-hydroxy-4-methoxy-phenyl group for a clear sweetness impression is also emphasized here, and furthermore the 2,6-dihydroxy-substitution pattern of the remaining aromatic compounds is assumed to be particularly important for a strong sweetness impression. No further sensory effects of compound (I) are described.

Compound (I) and other *ring-substituted* hesperetin dihydrochalcone are described in J. Agric. Food. Chem 1991, 39(1), 44-51 and their sweet intensity compared to a 6% aqueous sucrose solution wherein a similar or weaker sweet intensity for compound (I) is described. No further sensory effects of compound (I) are described.

J. Chem. Soc., Perkin Trans. 2 1998, 6, 1449-1454 describes a three-dimensional binding-site model for the structurally uncharacterised sweet-taste receptor, using pseudoreceptor modelling. The receptor model was derived based on 17 sweet compounds of the isovanillyl class (4-methoxy-3-hydroxybenzyl), inter alia compound (I), as the training set and consists of nine key amino-acid residues embedded in a hydrophobic receptor cavity. Quant. Struct.-Act. Relat. 2001, 20(1), 3-16 describes the results obtained by applying statistical models to develop QSARs for Isovanillyl derivatives. Compound (I) was here described, however compound (I) was not considered in the sensory evaluation.

It is important to mention that the literature referred to compound (I) the focus is drawn to the development of potential sweeteners. There is no hint to an effect of compound (I) in combination with sweet-modulating substances or other sweeteners as well as to an effect of compound (I) in combination with other fragrances or flavors (e.g. bitter-tasting substances).

In this context is merely known that the sweetness intensifying feature of dihydrochalcone-compounds differs from one to another.

In patent application WO 2007 107596 A1**,** 4-hydroxydihydrochalcones of Formula (7) and their salts are described for the intensification of sweet sensorial impressions. 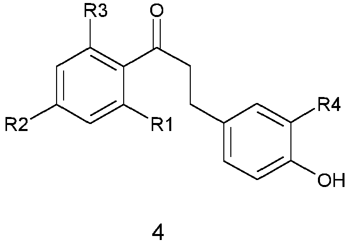 wherein R1, R2, R3 and R4 independently of one another denote H, OH or O-alkyl (with preferably 1-4 C-atoms, i.e. preferably C₁ to C₄ alkoxy), respectively, on condition that at least one of the residues R1, R2 or R3 signifies OH. However, for the sweetness-intensifying effects found here a 4-hydroxy-substitution was necessary.

It is particularly important to suppress an unpleasant taste impression, in particular a bitter taste impression, in many pharmaceutical active substances, since in this way the readiness of patients, in particular patients sensitive to a bitter taste such as children, to take the preparation orally, can be considerably increased. Many pharmaceutically active substances such as fluoroquinolone antibiotics, beta-lactam antibiotics, ambroxol, propylthiouracil [PROP], aspirin (acetyl salicylic acid), salicin, paracetamol (acetaminophene), ibuprofen, naproxen, ambroxol, guafenesin, omeprazole, pantoprazole, dextromorphane or quinine, to name but a few and for clarification purposes, have a pronounced bitter, astringent and/or metallic taste or aftertaste.

There is therefore a need to provide substances which in low concentrations effectively intensify sweet taste impressions of sweet substances, preferably the sweet taste impression of sugar-reduced foodstuffs and semi-luxury foods, in particular of sugar-reduced beverages such as soft drinks or protein shakes and semi-luxury foods with a low pH value, without adversely affecting the flavor profile.

### DESCRIPTION OF THE INVENTION

The object of the present invention is directed to an aroma composition comprising
(a) hesperetin dihydrochalcone (I) (3-(3-Hydroxy-4-methoxy-phenyl)-1-(2,4,6-trihydroxyphenyl)propan-1-one) 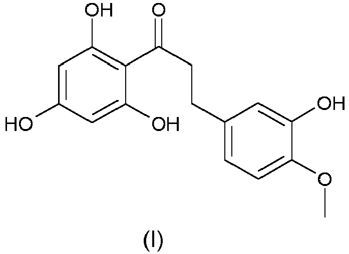 or a salt thereof, and
(b) high fructose corn syrup (HFCS).

Surprisingly, it has been observed that mixtures comprising hesperetin dihydrochalcone and High Fructose Corn Syrup (HFCS) provide improved sweetness at low concentrations and therefore are capable to substitute sucrose in oral compositions, particularly in beverages. The preparations thus obtained show similar sweetness and taste impressions at lower Brix values (which mean lower sugar content) and thus lower caloric intake.

### AROMA COMPOSITIONS

### Hesperetin dihydrochalcone.

For obtaining hesperetin dihydrochalcone its glycoside neohesperidin dihydrochalcone is heated in a mixture 2 M H₂SO₄ / MeOH (1:1) for some hours under reflux. After cooling to room temperature the reaction mixture was neutralized and filtrated. The obtained solid product is washed with water and dried to provide the product in form of white crystals.

### High Fructose Corn Syrup

High-fructose corn syrup (HFCS) (also called glucose-fructose, isoglucose and glucose-fructose syrup) is a sweetener made from corn starch that has been processed by glucose isomerase to convert some of its glucose into fructose. HFCS was first marketed in the early 1970s by the Clinton Corn Processing Company, together with the Japanese Agency of Industrial Science and Technology where the enzyme was discovered in 1965. As a sweetener, HFCS is often compared to granulated sugar, but manufacturing advantages of HFCS over sugar include that it is easier to handle and more cost-effective. The United States Food and Drug Administration have determined that HFCS is a safe ingredient for food and beverage manufacturing.

In the U.S., HFCS is among the sweeteners that mostly replaced sucrose (table sugar) in the food industry. Factors include production quotas of domestic sugar, import tariff on foreign sugar, and subsidies of U.S. corn, raising the price of sucrose and lowering that of HFCS, making it cheapest for many sweetener applications. The relative sweetness of HFCS 55, used most commonly in soft drinks, is comparable to sucrose. HFCS (and/or standard corn syrup) is the primary ingredient in most brands of commercial "pancake syrup", as a less expensive substitute for maple syrup. In recent times HFCS has become the most relevant sweetener for soft drinks.

HFCS is typically 24% water, the rest being mainly fructose and glucose with 0 to 5 wt.-percent unprocessed glucose oligomers. There are several varieties of HFCS, numbered by the percentage of fructose they contain encompassed by the present invention:
- HFCS 42 (≈ 42 wt.-percent fructose if water were removed) is used in beverages, processed foods, cereals, and baked goods;
- HFCS 55 is mostly used in soft drinks;
- HFCS 65 is used in soft drinks dispensed by soft drink machines.;
- HFCS 90 has some niche uses but is mainly mixed with HFCS 42 to make HFCS 55.

There are some related compositions of HFCS possible.

### Additional components

In a preferred embodiment the aroma compositions may comprise an additional sweetener or sweetness enhancer (component c) selected from the group consisting of steviosides, fructose, glucose, sugar alcohols (such as for example sorbitol or mannitol) saccharin, cyclamate, neohesperetin dihydrochalcone, erythritol, phloretin or a mixture thereof. Preferably the sweetener is a stevioside, more preferably rebaudioside A.

The compositions according to the present invention may include components (a) and (b) in a weight ratio of from about 0.5000 : 99.5000 to about 0.0001 : 99.9999 and preferably from about 0.0005:99.9995 to about 0.0500 : 99.9500.The composition may include components (a+b) and (c) in a weight ratio of from about 99.9995:0.0005 to about 25:75 and preferably 99.9950:0.0050 to about 99:1. The preferred range is particularly useful in case component (c) is rebaudioside A.

Preferably the composition of the present invention sugar content in the range of 0 to 13 °Bx, preferably 1 to 9 °Bx and more preferably 3 to 7 °Bx. Degrees Brix reflects the sugar content of an aqueous solution. One degree Brix is 1 gram of sucrose in 100 grams of solution and represents the strength of the solution as percentage by mass. If the solution contains dissolved solids other than pure sucrose, then the °Bx only approximates the dissolved solid content. The °Bx is traditionally used in the wine, sugar, carbonated beverage, fruit juice, maple syrup and honey industries and thus represents a value that is known for a person skilled in the art to which the present invention belongs.

The compositions according to the present invention may also encompass substances to mask or reduce an unpleasant taste impression, in particular a bitter taste impression; (component d) selected from the group consisting of homoeriodictyol or its sodium salts, eriodictyol, matairesinol, lariciresinol, naringenin, 5-hydroxy-4-(4-hydroxy-3-methoxyphenyl)-7-methoxychroman-2-one, 5,7-dihydroxy-4-(4-hydroxyphenyl)chroman-2-one, 5,7-dihydroxy-4-(4-hydroxy-3-methoxyphenyl)chroman-2-one and 2,4-dihydroxybenzoic acid, vanillyl amide and mixtures thereof.

Further substances to mask or reduce an unpleasant taste impression and/or to intensify a pleasant taste impression or taste correcting agents are - without limiting this invention to these - preferably selected from the group consisting of nucleotides (for example adenosine-5'-monophosphate, cytidine-5'-monophosphate) or the pharmaceutically acceptable salts thereof, lactisoles, sodium salts (for example sodium chloride, sodium lactate, sodium citrate, sodium acetate, sodium gluconoate), hydroxyflavanones, for example eriodictyol, sterubin (eriodictyol-7-methylether), homoeriodictyol, and the sodium, potassium, calcium, magnesium or zinc salts thereof (in particular those as described in EP 1258200 A1**,** which is part of this application by way of reference with respect to the corresponding compounds disclosed therein), hydroxybenzoic acid amides, for example 2,4-dihydroxybenzoic acid vanillylamide, 2,4-dihydroxybenzoic acid-N-(4-hydroxy-3-methoxybenzyl)amide, 2,4,6-trihydroxybenzoic acid-N-(4-hydroxy-3-methoxybenzyl)amide, 2-hydroxy-benzoic acid-N-4-(hydroxy-3-methoxybenzyl)amide, 4-hydroxybenzoic acid-N-(4-hydroxy-3-methoxybenzyl)amide, 2,4-dihydroxybenzoic acid-N-(4-hydroxy-3-methoxybenzyl)amide-mono-sodium salt, 2,4-dihydroxybenzoic acid-N-2-(4-hydroxy-3-methoxyphenyl)ethylamide, 2,4-dihydroxybenzoic acid-N-(4-hydroxy-3-ethoxybenzyl)amide, 2,4-dihydroxybenzoic acid-N-(3,4-dihydroxybenzyl)amide and 2-hydroxy-5-methoxy-N-[2-(4-hydroxy-3-methoxyphenyl)ethyl]amide; 4-hydroxybenzoic acid vanillylamide (in particular those as described in WO 2006/024587, which is part of this application by way of reference with respect to the corresponding compounds disclosed therein); hydroxydeoxybenzoins, for example 2-(4-hydroxy-3-methoxyphenyl)-1-(2,4,6-trihydroxyphenyl)ethanone, 1-(2,4-dihydroxyphenyl)-2-(4-hydroxy-3-methoxyphenyl)ethanone, 1-(2-hydroxy-4-methoxyphenyl)-2-(4-hydroxy-3-methoxyphenyl)ethanone) (in particular those as described in WO 2006 106023 A1which is part of this application by way of reference with respect to the corresponding compounds disclosed therein); hydroxyphenyl alkane diones, for example gingerdione-[2], gingerdione-[3], gingerdione-[4], dehydrogingerdione-[2], dehydrogingerdione-[3], dehydrogingerdione-[4]) (in particular those as described in WO 2007 003527 A1 which is part of this application by way of reference with respect to the corresponding compounds disclosed therein); diacetyl trimers (in particular those as described in WO 2006 058893 A1 which is part of this application by way of reference with respect to the corresponding compounds disclosed therein); gamma-aminobutyric acids (in particular those as described in WO 2005 096841 A1 which is part of this application by way of reference with respect to the corresponding compounds disclosed therein); divanillins (in particular those as described in WO 2004 078302 A1 which is part of this application by way of reference with respect to the corresponding compounds disclosed therein) and 4-hydroxydihydrochalcones (preferably as described in US 20080227867 A1**,** which is part of this application by way of reference with respect to the corresponding compounds disclosed therein), in this respect in particular phloretin and davidigenin, amino acids or mixtures of whey proteins with lecithins, hesperetin as disclosed in WO 2007014879 which is part of this application by way of reference with respect to the corresponding compounds, 4-hydroxychalcones as disclosed in WO 2007 107596 A1 which is part of this application by way of reference with respect to the corresponding compounds, or propylene phenyl glycosides (chavicolgylcosides) as described in EP 1955601 A1 which is part of this application by way of reference with respect to the corresponding compounds, pellitorin and derived flavoring compositions, umami compounds as described in WO 2008 046895 A1 and EP 1989944 A1 which are in each case part of this application by way of reference with respect to the corresponding compounds; matairesinol and neoflavanoide as described in WO 2012 146584 A1**,** US 2013 078192 A1 and US 2013 084252 A1**,** neoisoflavonoids as described in EP 2570035 A1**,** EP 2725026 A1 **or** EP 2570036 A1**.**

The aroma compositions according to the invention comprising hesperetin dihydrochalcone (I) and/or its salts and HFCS, are preferably manufactured in that the mixtures are incorporated with a solid or liquid excipient in the form of a solution or a mixture in a corresponding preparation, i.e. in particular serving for nutrition, as a food supplement, for oral care or pleasure. As a solution these preparations according to the invention can advantageously also be converted by spray drying into a solid preparation.

According to a further preferred embodiment, for manufacturing preparations according to the invention hesperetin dihydrochalcone (I) according to the invention and/or its salts, HFCS and if necessary further ingredients of the preparation according to the invention can first (i.e. prior to incorporation in the preparation) be incorporated in emulsions, in liposomes, e.g. starting from phosphatidylcholine, in microspheres, in nanospheres or also in capsules, granules or extrudates in a matrix suitable for foodstuffs and semi-luxury foods, e.g. starch, starch derivatives, cellulose or cellulose derivatives (e.g. hydroxypropylcellulose), other polysaccharides (e.g. alginate), natural fats, natural waxes (e.g. beeswax, carnauba wax) or proteins, e.g. gelatine.

In a further preferred manufacturing method hesperetin dihydrochalcone (I) and/or its salts are first complexed with a plurality of suitable complexing agents, for example with cyclodextrins or cyclodextrin derivatives, preferably α- or β-cyclodextrin, and used in this complexed form.

Particular preference is for a preparation according to the invention in which the matrix is selected such that the hesperetin dihydrochalcone (I) and/or the salt(s) of the hesperetin dihydrochalcone (I) and HFCS have an improved sugar-like taste while less calorie intake.

### ORAL COMPOSITIONS

Another object of the present invention refers to an oral composition comprising the aroma composition described above. Oral compositions according to the invention are any preparations or compositions which are suitable for consumption and are used for nutrition or enjoyment purposes, and are generally products which are intended to be introduced into the human or animal oral cavity, to remain there for a certain time and then either be eaten (e.g. ready-to-eat oral stuffs or feeds, see also herein below) or removed from the oral cavity again (e.g. chewing gums).

Such products include any substances or products which in the processed, partially processed or unprocessed state are to be ingested by humans or animals. They also include substances which are added to orally consumable products during their manufacture, preparation or treatment and which are intended to be introduced into the human or animal oral cavity.

Typically said oral compositions contain the aroma compositions in an amount of from 0.1 to about 30 wt.-percent, preferably from about 0.5 to 25 wt.-percent and more preferably from about 1 to about 18 wt.-percent.

The oral compositions according to the invention also include substances which in the unchanged, treated or prepared state are to be swallowed by a human or animal and then digested; in this respect, the orally consumable products according to the invention also include casings, coatings or other encapsulations which are to be swallowed at the same time or which may be expected to be swallowed. The expression "orally consumable product" covers ready-to-eat oral stuffs and feeds, that is to say oral stuffs or feeds that are already complete in terms of the substances that are important for the taste. The expressions "ready-to-eat oral stuff and "ready-to-eat feed" also include drinks as well as solid or semi-solid ready-to-eat oral stuffs or feeds. Examples which may be mentioned are frozen products, which must be thawed and heated to eating temperature before they are eaten. Products such as yoghurt or ice-cream as well as chewing gums or hard caramels are also included among the ready-to-eat oral stuffs or feeds.

Preferred oral compositions according to the invention also include "semi-finished products". Within the context of the present text, a semi-finished product is to be understood as being an orally consumable product which, because of a very high content of flavorings and taste-imparting substances, is unsuitable for use as a ready-to-eat orally consumable product (in particular oral stuff or feed). Only by mixing with at least one further constituent (e.g. by reducing the concentration of the flavorings and taste-imparting substances in question) and optionally further process steps (e.g. heating, freezing) is the semi-finished product converted into a ready-to-eat orally consumable product (in particular oral stuff or feed). Oral composition according to the invention preferably comprises one or more preparations for nutrition or enjoyment purposes. These include in particular (reduced-calorie) baked goods (e.g. bread, dry biscuits, cakes, other baked articles), confectionery (e.g. chocolates, chocolate bars, other products in bar form, fruit gums, dragees, hard and soft caramels, chewing gum), non-alcoholic drinks (e.g. cocoa, coffee, green tea, black tea, (green, black) tea drinks enriched with (green, black) tea extracts, rooibos tea, other herbal teas, fruit-containing soft drinks, isotonic drinks, refreshing drinks, nectars, fruit and vegetable juices, fruit or vegetable juice preparations), instant drinks (e.g. instant cocoa drinks, instant tea drinks, instant coffee drinks), meat products (e.g. ham, fresh sausage or raw sausage preparations, spiced or marinated fresh or salt meat products), eggs or egg products (dried egg, egg white, egg yolk), cereal products (e.g. breakfast cereals, muesli bars, precooked ready-to-eat rice products), dairy products (e.g. full-fat or reduced-fat or fat-free milk drinks, rice pudding, yoghurt, kefir, cream cheese, soft cheese, hard cheese, dried milk powder, whey, butter, buttermilk, partially or completely hydrolyzed milk-protein-containing products), products made from soy protein or other soybean fractions (e.g. soy milk and products produced therefrom, drinks containing isolated or enzymatically treated soy protein, drinks containing soy flour, preparations containing soy lecithin, fermented products such as tofu or tempeh or products produced therefrom and mixtures with fruit preparations and optionally flavors), fruit preparations (e.g. jams, sorbets, fruit sauces, fruit fillings), vegetable preparations (e.g. ketchup, sauces, dried vegetables, frozen vegetables, precooked vegetables, boiled-down vegetables), snacks (e.g. baked or fried potato crisps or potato dough products, maize- or groundnut-based extrudates), fat- and oil-based products or emulsions thereof (e.g. mayonnaise, remoulade, dressings, in each case full-fat or reduced-fat), other ready-made dishes and soups (e.g. dried soups, instant soups, precooked soups), spices, spice mixtures and in particular seasonings which are used, for example, in the snacks field, sweetener preparations, tablets or sachets, other preparations for sweetening or whitening drinks or other orals. The preparations within the scope of the invention can also be used in the form of semi-finished products for the production of further preparations for nutrition or enjoyment purposes. The preparations within the scope of the invention can also be in the form of capsules, tablets (uncoated and coated tablets, e.g. enteric coatings), dragees, granules, pellets, solids mixtures, dispersions in liquid phases, in the form of emulsions, in the form of powders, in the form of solutions, in the form of pastes, or in the form of other preparations which can be swallowed or chewed, and in the form of oral supplements.

The preparations can also be in the form of capsules, tablets (uncoated and coated tablets, e.g. enteric coatings), dragees, granules, pellets, solids mixtures, dispersions in liquid phases, in the form of emulsions, in the form of powders, in the form of solutions, in the form of pastes, or in the form of other preparations which can be swallowed or chewed, for example in the form of oral supplements.

The semi-finished products are generally used for the production of ready-to-use or ready-to-eat preparations for nutrition or enjoyment purposes.

Further constituents of a ready-to-eat preparation or semi-finished product for nutrition or enjoyment purposes can be conventional base substances, auxiliary substances and additives for orals or enjoyment orals, for example water, mixtures of fresh or processed, vegetable or animal base or raw substances (e.g. raw, roast, dried, fermented, smoked and/or boiled meat, bone, cartilage, fish, vegetables, herbs, nuts, vegetable juices, vegetable pastes or mixtures thereof), digestible or non-digestible carbohydrates (e.g. sucrose, maltose, fructose, glucose, dextrins, amylose, amylopectin, inulin, xylans, cellulose, tagatose), sugar alcohols (e.g. sorbitol, erythritol), natural or hardened fats (e.g. tallow, lard, palm fat, cocoa fat, hardened vegetable fat), oils (e.g. sunflower oil, groundnut oil, maize germ oil, olive oil, fish oil, soya oil, sesame oil), fatty acids or their salts (e.g. potassium stearate), proteinogenic or non-proteinogenic amino acids and related compounds (e.g. γ-aminobutyric acid, taurine), peptides (e.g. glutathione), natural or processed proteins (e.g. gelatin), enzymes (e.g. peptidases), nucleic acids, nucleotides, taste correctors for unpleasant taste impressions, further taste modulators for further, generally not unpleasant taste impressions, other taste-modulating substances (e.g. inositol phosphate, nucleotides such as guanosine monophosphate, adenosine monophosphate or other substances such as sodium glutamate or 2-phenoxypropionic acid), emulsifiers (e.g. lecithins, diacylglycerols, gum arabic), stabilisers (e.g. carrageenan, alginate), preservatives (e.g. benzoic acid and its salts, sorbic acid and its salts), antioxidants (e.g. tocopherol, ascorbic acid), chelators (e.g. citric acid), organic or inorganic acidifying agents (e.g. acetic acid, phosphoric acid), additional bitter substances (e.g. quinine, caffeine, limonene, amarogentine, humulone, lupulone, catechols, tannins), substances that prevent enzymatic browning (e.g. sulfite, ascorbic acid), ethereal oils, plant extracts, natural or synthetic colorings or coloring pigments (e.g. carotinoids, flavonoids, anthocyans, chlorophyll and derivatives thereof), spices, trigeminally active substances or plant extracts containing such trigeminally active substances, synthetic, natural or nature-identical flavorings or odorants as well as odour correctors.

Oral compositions according to the invention, for example those in the form of preparations or semi-finished products, preferably comprise a flavor composition in order to complete and refine the taste and/or odour. A preparation can comprise as constituents a solid carrier and a flavor composition. Suitable flavor compositions comprise, for example, synthetic, natural or nature-identical flavorings, odorants and taste-imparting substances, reaction flavorings, smoke flavorings or other flavor-giving preparations (e.g. protein (partial) hydrolysates, preferably protein (partial) hydrolysates having a high arginine content, barbecue flavorings, plant extracts, spices, spice preparations, vegetables and/or vegetable preparations) as well as suitable auxiliary substances and carriers. Particularly suitable here are the flavor compositions or constituents thereof which produce a roasted, meaty (in particular chicken, fish, seaoral, beef, pork, lamb, mutton, goat), vegetable-like (in particular tomato, onion, garlic, celery, leek, mushroom, aubergine, seaweed), spicy (in particular black and white pepper, cardamom, nutmeg, pimento, mustard and mustard products), fried, yeast-like, boiled, fatty, salty and/or pungent flavor impression and accordingly can enhance the spicy impression. The flavor compositions generally comprise more than one of the mentioned ingredients.

The oral compositions of the present invention are preferably selected from the group comprising
- confectionery, preferably reduced-calorie or calorie-free confectionery, preferably selected from the group comprising muesli bar products, fruit gums, dragees, hard caramels and chewing gum,
- non-alcoholic drinks, preferably selected from the group comprising green tea, black tea, (green, black) tea drinks enriched with (green, black) tea extracts, rooibos tea, other herbal teas, low-sugar or sugar-free soft drinks with or without fruit content, isotonic drinks, nectars, fruit and vegetable juices, fruit and vegetable juice preparations,
- instant drinks, preferably selected from the group comprising instant (green, black, rooibos, herbal) tea drinks,
- cereal products, preferably selected from the group comprising low-sugar and sugar-free breakfast cereals and muesli bars,
- dairy products, preferably selected from the group comprising reduced-fat and fat-free milk drinks, yoghurt, kefir, whey, buttermilk and ice-cream,
- products made from soy protein or other soybean fractions, preferably selected from the group comprising soy milk, products produced from soy milk, drinks containing isolated or enzymatically treated soy protein, drinks containing soy flour, preparations containing soy lecithin, products produced from preparations containing soy lecithin and mixtures with fruit preparations and optionally flavors,
- sweetener preparations, tablets and sachets,
- sugar-free dragees,
- ice-cream, with or without milk-based constituents, preferably sugar-free.
- The preferred oral compositions represent beverages such as for example sparkling and non-sparkling soft drinks or protein shakes.

The oral compositions according to the present invention may require the presence of further additives, such as for example additional aroma or flavoring compounds, additional sweeteners or sweetness enhancers, thickeners, physiological cooling agents, acidifiers or vitamins, which are illustrated in the following sections. The disclosure of additives may overlap with compounds already mentioned among the groups (c) and (d).

### Additional aroma or flavoring compounds

Aroma compounds and flavoring agents (component d) are well known in the art can be added to the flavor compositions of the invention. These flavoring agents can be chosen from synthetic flavoring liquid and/or oils derived from plants leaves, flowers, fruits and so forth, and combinations thereof. Representative flavoring liquids include: artificial, natural or synthetic fruit flavors such as eucalyptus, lemon, orange, banana, grape, lime, apricot and grapefruit oils and fruit essences including apple, strawberry, cherry, orange, pineapple and so forth; bean and nut derived flavors such as coffee, cocoa, cola, peanut, almond and so forth; and root derived flavors such as licorice or ginger.

The flavoring agent is preferably selected from the group consisting of essential oils and extracts, tinctures and balsams, such as, for example, anisole, basil oil, bergamot oil, bitter almond oil, camphor oil, citronella oil, lemon oil; Eucalyptus citriodora oil, eucalyptus oil, fennel oil, grapefruit oil, camomile oil, spearmint oil, caraway oil, lime oil, mandarin oil, nutmeg oil (in particular nutmeg blossom oil = maces oil, mace oil), myrrh oil, clove oil, clove blossom oil, orange oil, oregano oil, parsley (seed) oil, peppermint oil, rosemary oil, sage oil (clary sage, Dalmatian or Spanish sage oil), star aniseed oil, thyme oil, vanilla extract, juniper oil (in particular juniper berry oil), wintergreen oil, cinnamon leaf oil; cinnamon bark oil, and fractions thereof, or constituents isolated therefrom.

It is of particular advantage if the flavored composition according to the invention comprises at least one flavoring agent, preferably two, three, four, five, six, seven, eight or more flavoring agents chosen from the following group: menthol (preferably l-menthol and/or racemic menthol), anethole, anisole, anisaldehyde, anisyl alcohol, (racemic) neomenthol, eucalyptol (1,8-cineol), menthone (preferably L-menthone), isomenthone (preferably D-isomenthone), isopulegol, menthyl acetate (preferably L-menthyl acetate), menthyl propionate, carvone (preferably (-)-carvone, optionally as a constituent of a spearmint oil), methyl salicylate (optionally as a constituent of a wintergreen oil), eugenol acetate, isoeugenol methyl ether, beta-homocyclocitral, eugenol, isobutyraldehyde, 3-octanol, dimethyl sulfide, hexanol, hexanal, trans-2-hexenal, cis-3-hexenol, 4-terpineol, piperitone, linalool, 8-ocimenyl acetate, isoamyl alcohol, isovaleraldehyde, alpha-pinene, beta-pinene, limonene (preferably D-limonene, optionally as a constituent of an essential oil), piperitone, trans-sabinene hydrate, menthofuran, caryophyllene, germacrene D, cinnamaldehyde, mint lactone, thymol, gamma-octalactone, gamma-nonalactone, gamma-decalactone, (1,3E,5Z)-undecatriene, 2-butanone, ethyl formate, 3-octyl acetate, isoamyl isovalerate, cis- and trans-carvyl acetate, p-cymol, damascenone, damascone, cis-rose oxide, transrose oxide, fenchol, acetaldehyde diethyl acetal, 1-ethoxyethyl acetate, cis-4-heptenal, cisjasmone, methyl dihydrojasmonate, 2'-hydroxypropiophenone, menthyl methyl ether, myrtenyl acetate, 2-phenylethyl alcohol, 2-phenylethyl isobutyrate, 2-phenylethyl isovalerate, geraniol, nerol and viridiflorol.

In particular preferred aroma or flavoring compounds encompass menthol, cineol, eugenol, thymol, cinnamic aldehyde, peppermint oil, spearmint oil, eucalyptus oil, thyme oil, cinnamon oil, clove oil, spruce needle oil, fennel oil, sage oil, aniseed oil, star anise oil, chamomile oil, and caraway oil, and their mixtures.

### Additional sweeteners

The term "sweeteners" here denotes substances having a relative sweetening power of at least 25, based on the sweetening power of sucrose (which accordingly has a sweetening power of 1). Sweeteners to be used in an orally consumable product (in particular oralstuff, feed or medicament) according to the invention (a) are preferably non-cariogenic and/or have an energy content of not more than 5 kcal per gram of the orally consumable product.

**Naturally occurring sweeteners,** preferably selected from the group consisting of miraculin, monellin, mabinlin, thaumatin, curculin, brazzein, pentaidin, D-phenylalanine, D-tryptophan, and extracts or fractions obtained from natural sources, comprising those amino acids and/or proteins, and the physiologically acceptable salts of those amino acids and/or proteins, in particular the sodium, potassium, calcium or ammonium salts; neohesperidin dihydrochalcone, naringin dihydrochalcone, stevioside, steviolbioside, rebaudiosides, in particular rebaudioside A, rebaudioside B, rebaudioside C, rebaudioside D, rebaudioside E, rebaudioside F, rebaudioside G, rebaudioside H, dulcosides and rubusoside, suavioside A, suavioside B, suavioside G, suavioside H, suavioside I, suavioside J, baiyunoside 1, baiyunoside 2, phlomisoside 1, phlomisoside 2, phlomisoside 3 and phlomisoside 4, abrusoside A, abrusoside B, abrusoside C, abrusoside D, cyclocaryoside A and cyclocaryoside I, osladin, polypodoside A, strogin 1, strogin 2, strogin 4, selligueain A, dihydroquercetin 3-acetate, perillartin, telosmoside A₁₅, periandrin I-V, pterocaryosides, cyclocaryosides, mukuroziocides, trans-anethole, trans-cinnamaldehyde, bryosides, bryonosides, bryonodulcosides, carnosiflosides, scandenosides, gypenosides, trilobatin, phloridzin, dihydroflavanols, hematoxylin, cyanin, chlorogenic acid, albiziasaponin, telosmosides, gaudichaudioside, mogrosides, mogroside V, hernandulcins, monatin, phyllodulcin, glycyrrhetinic acid and derivatives thereof, in particular glycosides thereof such as glycyrrhizine, and the physiologically acceptable salts of those compounds, in particular the sodium, potassium, calcium or ammonium salts;

extracts or concentrated fractions of the extracts, selected from the group comprising thaumatococcus extracts (katamfe plant), extracts from *Stevia* ssp. (in particular *Stevia rebaudiana),* swingle extracts *(Momordica* or *Siratia grosvenorii,* Luo-Han-Guo), extracts from *Glycerrhyzia* ssp. (in particular *Glycerrhyzia glabra),* extracts from *Rubus* ssp. (in particular *Rubus suavissimus),* citrus extracts and extracts from *Lippia dulcis;*

**Synthetic sweet-tasting substances,** preferably selected from the group comprising magap, sodium cyclamate or other physiologically acceptable salts of cyclamic acid, acesulfame K or other physiologically acceptable salts of acesulfame, neohesperidin dihydrochalcone, naringin dihydrochalcone, saccharin, saccharin sodium salt, aspartame, superaspartame, neotame, alitame, advantame, perillartin, sucralose, lugduname, carrelame, sucrononate and sucrooctate.

### Thickeners

Advantageous thickeners in a preferred orally consumable product (in particular oral stuff, feed or medicament) according to the invention are selected from the group comprising: crosslinked polyacrylic acids and derivatives thereof, polysaccharides and derivatives thereof, such as xanthan gum, agar-agar, alginates or tyloses, cellulose derivatives, for example carboxymethylcellulose or hydroxycarboxymethylcellulose, fatty alcohols, monoglycerides and fatty acids, polyvinyl alcohol and polyvinylpyrrolidone.

Preference is given according to the invention to an orally consumable product (in particular oral stuff or feed) which comprises milk thickened with lactic acid bacteria and/or cream thickened with lactic acid bacteria and which preferably is selected from the group comprising yoghurt, kefir and quark.

A oral composition according to the invention comprising milk thickened with lactic acid bacteria and/or cream thickened with lactic acid bacteria is advantageously an orally consumable product which comprises a probiotic, wherein the probiotic is preferably selected from the group comprising Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) and Lactobacillus rhamnosus (ATCC 53013).

### Physiological cooling agents

The compositions may also contain one or more substances with a physiological cooling effect (cooling agents), which are preferably selected here from the following list: menthol and menthol derivatives (for example L-menthol, D-menthol, racemic menthol, isomenthol, neoisomenthol, neomenthol) menthylethers (for example (l-menthoxy)-1,2-propandiol, (I-menthoxy)-2-methyl-1,2-propandiol, I-menthyl-methylether), menthone glyceryl acetal, menthone glyceryl ketal or mixtures of both, menthylesters (for example menthylformiate, menthylacetate, menthylisobutyrate, menthyhydroxyisobutyrat, menthyl-lactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcar-bonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{α}-(menthanecarbonyl)glycinethylester [WS5], as described in US 4,150,052**,** menthanecarboxylic acid-N-(4-cyanophenyl)amide or menthanecarboxylic acid-N-(4-cyanomethylphenyl)amide as described in WO 2005 049553 A1**,** menthanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (l-(-)-isopulegol, l-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxy-phenyl)-5-methyl-2-(1-isopropyl)cyclohexane-carboxamide [WS12], oxamates (preferably those described in EP 2033688 A2**)** and [(1R,2S,5R)-2-isopropyl-5-methyl-cyclohexyl] 2-(ethylamino)-2-oxo-acetate (X Cool).

### Acidifiers and acid regulators

Suitable **acidifiers** capable of adjusting the oral composition to a pH value of less than 7 include:
- E 260: - Acet ic acid
- E 270: - Lactic acid
- E 290: - Carbondioxide
- E 296: - Malic acid
- E 297: - Fumaric Acid
- E 330: - Citric acid
- E 331: - sodium citrate
- E 332: - potassium citrate
- E 333: - Calcium citrate
- E 334: - Tartaric acid
- E 335: - Sodium tartrate
- E 336: - Potassium tartrate
- E 337: - Sodium/Potassium tartrate
- E 338: - Phosphoric acid
- E 353: - Meta malic acid
- E 354: - Calcium tartrate
- E 355: - Adipic acid
- E 363: - Succinic acid
- E 380: - Triammonium citrate
- E 513: - Sulfuric acid
- E 574: - Gluconic acid
- E 575: - Glucono-delta-Lactone

**Acid regulators** are aditives capable of keeping pH value of the composition constant. Basically these compounds acta s buffers. Suitable examples encompass:
- E 170: - Calcium carbonate
- E 260-263: - Acetic acid and its salts
- E 270: - Lactic acid
- E 296: - Malic acids and its salts
- E 297: - Fumaric acid
- E 325-327: - Lactates
- E 330-333: - Citric acid and its salts
- E 334-337: - Tartaric acid and its salts
- E 339-341: - Orthophosphates
- E 350-352: - Maleic acid and its salts
- E 450-452: - Di-, Tri- und Polyphosphates
- E 500-504: - Carbonates
- E 507: - HCI and hlorides
- E 513-517: - Sulfuric acid and its salts
- E 524-528: - Hydroxides
- E 529-530: - Oxides
- E 355-357: - Adipic acid and its salts
- E 574-578: - Gluconic acid and its salts

### Vitamins

In another embodiment of the present invention the compositions may include vitamins (component e1). Vitamins have diverse biochemical functions. Some have hormone-like functions as regulators of mineral metabolism (e.g., vitamin D), or regulators of cell and tissue growth and differentiation (e.g., some forms of vitamin A). Others function as antioxidants (e.g., vitamin E and sometimes vitamin C). The largest numbers of vitamins (e.g. B complex vitamins) act as precursors for enzyme cofactors that help enzymes in their work as catalysts in metabolism. In this role, vitamins may be tightly bound to enzymes as part of prosthetic groups: For example, biotin is part of enzymes involved in making fatty acids. Vitamins may also be less tightly bound to enzyme catalysts as coenzymes, detachable molecules that function to carry chemical groups or electrons between molecules. For example, folic acid carries various forms of carbon group - methyl, formyl, and methylene - in the cell. Although these roles in assisting enzyme-substrate reactions are vitamins' best-known function, the other vitamin functions are equally important. In the course of the present invention suitable vitamins are selected from the group consisting of
- Vitamin A (retinol, retinal, beta carotene),
- Vitamin B₁ (thiamine),
- Vitamin B₂ (riboflavin),
- Vitamin B₃ (niacin, niacinamide),
- Vitamin B₅ (panthothenic acid),
- Vitamin B₆ (pyridoxine, pyridoxamine, paridoxal),
- Vitamin B₇ (biotin),
- Vitamin B₉ (folic acid, folinic acid),
- Vitamin B₁₂ (cyanobalamin, hydoxycobalmin, methylcobalmin),
- Vitamin C (ascorbic acid),
- Vitamin D (cholecalciferol),
- Vitamin E (tocopherols, tocotrienols), and
- Vitamin K (phyolloquinone, menaquinone).

The preferred vitamins are ascorbic acid and tocopherols. Said vitamins may be present in the food composition in amounts of about 0.1 to about 5 % b.w., and preferably about 0.5 to about 1 % b.w.

In the following typical oral compositions are illustrated in more detail:

### BEVERAGES

Beverages covered by the present invention include alcoholic and non-alcoholic types, sparkling and non-sparkling drinks, in particular soft drinks including ice teas and protein shakes. Their main component is of course water, optionally carbonated water. Further major components are flavors and aromas and sweeteners and acidulents, preferably citric acid and/or phosphoric acid.

### CHEWING GUMS

Chewing gums typically consist of a water- insoluble vase component, a water-soluble component and additives providing for example a specific flavor.

The water-insoluble base, which is also known as the "gum base", typically comprises natural or synthetic elastomers, resins, fats and oils, plasticizers, fillers, softeners, dyes and optionally waxes. The base normally makes up 5 to 95% by weight, preferably 10 to 50% by weight and more particularly 20 to 35% by weight of the composition as a whole. In one typical embodiment of the invention, the base consists of 20 to 60% by weight synthetic elastomers, 0 to 30% by weight natural elastomers, 5 to 55% by weight plasticizers, 4 to 35% by weight fillers, 5 to 35% by weight softeners and small amounts of additives, such as dyes, antioxidants and the like, with the proviso that they are soluble in water at best in small quantities.

Suitable synthetic elastomers are, for example, polyisobutylenes with average molecular weights (as measured by GPC) of 10,000 to 100,000 and preferably 50,000 to 80,000, isobutylene/isoprene copolymers ("butyl elastomers"), styrene/butadiene copolymers (styrene:butadiene ratio, for example, 1:3 to 3:1). polyvinyl acetates with average molecular weights (as measured by GPC) of 2,000 to 90,000 and preferably 10,000 to 65,000, polyisoprenes, poly-ethylenes, vinyl acetate/vinyl laurate copolymers and mixtures thereof. Examples of suitable natural elastomers are rubbers, such as for example smoked or liquid latex or guayuls, and natural gums, such as jelutong, lechi caspi, perillo, sorva, massaranduba balata, massaranduba chocolate, nispero, rosindinba, chicle, gutta hang kang and mixtures thereof. The choice of the synthetic and natural elastomers and their mixing ratios essentially depends on whether or not bubbles are to be produced with the chewing gums (bubble gums). Elastomer mixtures containing jelutong, chicle, sorva and massaranduba are preferably used.

In most cases, the elastomers are too hard or lack plasticity for satisfactory processing, so that it has been found to be of advantage to use special plasticizers which, of course, must also satisfy in particular all requirements relating to acceptability as food additives. In this respect, suitable plasticizers are, above all, esters of resin acids, for example esters of lower aliphatic alcohols or polyols with completely or partly hydrogenated, monomeric or oligomeric resin acids. In particular, the methyl, glycerol or pentaerythritol esters or mixtures thereof are used for this purpose. Alternatively, terpene resins, which may be derived from .alpha.-pinene, .beta.-pinene, .delta.-limonene or mixtures thereof, could also be used.

Suitable fillers or texturizers are magnesium or calcium carbonate, ground pumice stone, silicates, especially magnesium or aluminium silicates, clays, aluminium oxides, talcum, titanium dioxide, mono-, di- and tricalcium phosphate and cellulose polymers.

Suitable softeners or emulsifiers are tallow, hydrogenated tallow, hydrogenated or partly hydrogenated vegetable oils, cocoa butter, partial glycerides, lecithin, triacetin and saturated or unsaturated fatty acids containing 6 to 22 and preferably 12 to 18 carbon atoms and mixtures thereof.

Suitable dyes and whiteners are, for example, the FD&C types, plant and fruit extracts permitted for coloring foods and titanium dioxide. The gum bases may also contain waxes or may be wax-free

In addition to the water-insoluble gum base, chewing gum preparations regularly contain a water-soluble component which is formed, for example, by softeners, sweeteners, fillers, flavors, flavor enhancers, emulsifiers, dyes, acidifiers, antioxidants and the like, with the proviso that the constituents have at least adequate solubility in water. Accordingly, individual constituents may belong both to the water-insoluble phase and to the water-soluble phase, depending on the water solubility of the special representatives. However, combinations may also be used, for example a combination of a water-soluble and a water-insoluble emulsifier, in which case the individual representatives are present in different phases. The water-insoluble component usually makes up 5 to 95% by weight and preferably 20 to 80% by weight of the preparation.

Water-soluble softeners or plasticizers are added to the chewing gum compositions to improve chewability and the chewing feel and are present in the mixtures in quantities of typically 0.5 to 15% by weight. Typical examples are glycerol, lecithin and aqueous solutions of sorbitol, hydrogenated starch hydrolysates or corn sirup.

Fillers are particularly suitable for the production of low-calorie chewing gums and may be selected, for example, from polydextrose, raftilose, raftilin, fructo-oligosaccharides (NutraFlora), palatinose oligosaccharides, guar gum hydrolyzates (Sun Fiber) and dextrins.

The chewing gums may additionally contain auxiliaries and additives which are suitable, for example, for dental care, more particularly for controlling plaque and gingivitis, such as for example chlorhexidine, CPC or triclosan. They may also contain pH adjusters (for example buffer or urea), anti-caries agents (for example phosphates or fluorides), biogenic agents (antibodies, enzymes, caffeine, plant extracts), providing these substances are permitted in foods and do not undesirably interact with one another.

### INDUSTRIAL APPLICATION

Another object of the present invention is related to a method for providing an oral composition, particularly a beverage with high sweetness and reduced calories, encompassing the following steps:
(i) providing a base for the oral composition or the beverage respectively;
(ii) providing the aroma composition as described above,
(iii) blending both compounds, and optionally
(iv) adding carbonic acid to the formulation.

The term "base" relates to the oral composition or beverage and its ingredients as described above.

Another object of the present invention to the use of the aroma composition as described above for sweetening a food composition and/or as a substituent for caloric sweeteners.

With regard to preferred mixtures, preferred ingredients and preferred ranges reference is made to the disclosures provided above. All the preferences apply also to the method and uses; therefore, repeating them is not necessary.

The invention is illustrated in more detail by the following examples, however, without limiting the invention to them.

### EXAMPLES

### MANUFACTURING AND APPLICATION EXAMPLES

### EXAMPLE M1

### Synthesis of 3-(3-Hydroxy-4-methoxy-phenyl)-1-(2,4,6-trihydroxyphenyl)propan-1-one (hesperetin dihydrochalcone (I))

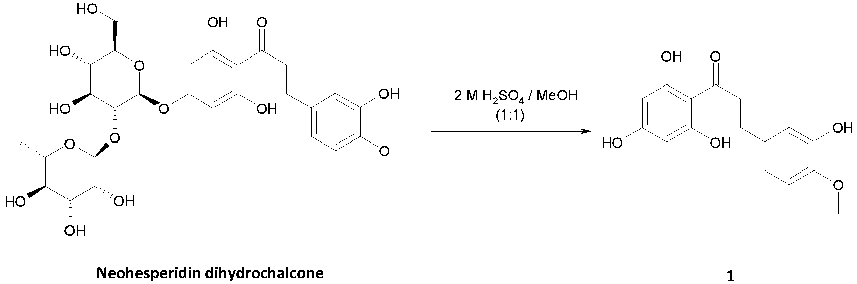

Neohesperidin dihydrochalcone in a mixture 2 M H₂SO₄/ MeOH (1:1) was heated for 6 hours under reflux. After cooling to room temperature the reaction mixture was neutralized by addition of 2M NaOH and filtrated. The obtained solid product was washed with water (3x) and dried at 40 °C under vacuum to afford hesperetin dihydrochalcone (I) as a white solid product (Yield: 77%).

¹H-NMR (400 MHz, DMSO-*d*₆): *δ* = 12.25 (s, 2H), 10.36 (s, 1H), 8.79 (s, 1H), 6.80 (d, *J* = 8.2 Hz, 1H), 6.66 (d, *J* = 2.1 Hz, 1H), 6.59 (dd, *J* = 8.2, 2.2 Hz, 1H), 5.81 (s, 2H), 3.72 (s, 3H), 3.22 (dd, *J* = 8.5, 6.9 Hz, 2H), 2.74 (dd, *J* = 8.5, 6.9 Hz, 2H).

¹³C NMR (101 MHz, DMSO-*d*₆): *δ*= 203.97, 164.51,164.12 (2C), 146.19, 145.70, 134.13, 118.59, 115.61, 112.24, 103.60, 94.54 (2C), 55.59, 45.11, 29.42.

### EXAMPLES 1-8, COMPARISON EXAMPLES C1-C4

### Carbonated low-caloric drinks

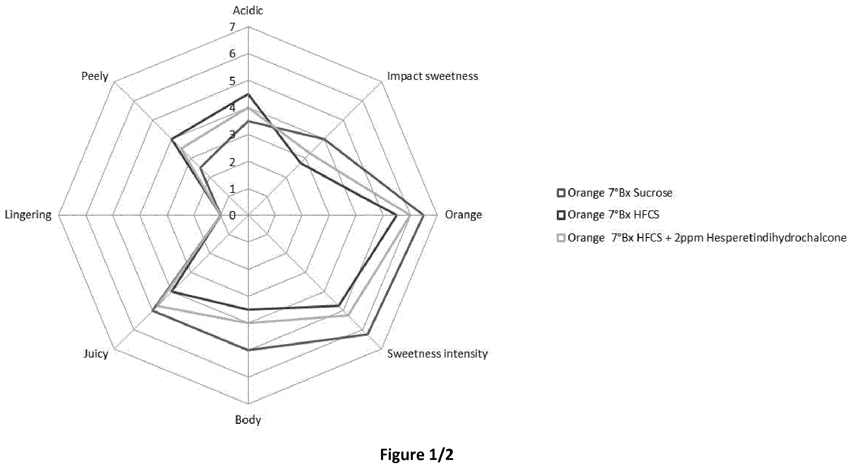

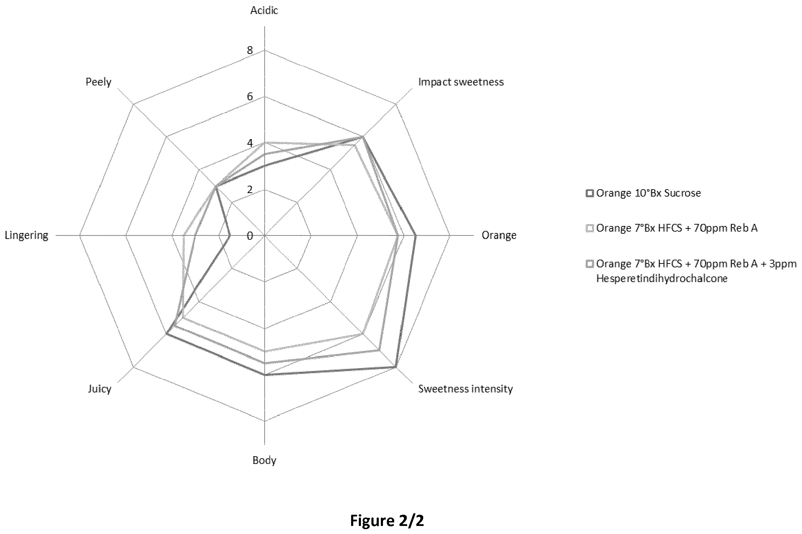

The solid components or ingredients are individually mixed with water, combined and made up to 100 g with water. The concentrate obtained is then allowed to age over night at ambient temperature. Finally, 1 part concentrate is mixed with (carbonated) water. Subsequently taste impressions of the compositions were determined by a panel of 6 experienced individuals on a scale (1) = low to (7) = very strong. The results are provided in **Table 1 and Table 2.** Examples 1 to 8 are according to the invention, Examples C1 to C4 serve for comparison. The results for Table 1 are also depicted in a spider-net diagram according to **Figure 1****.** **Figures 2** show similar results for ternary mixtures of hesperetin dichydrochalcone, HFCS and Rebaudioside A.

**Table 1**

| Non-carbonated low-caloric soft drinks (all amounts in wt.-percent) | | | | | | |
|---|---|---|---|---|---|---|
| **Ingredient** | **C1** | **C2** | **1** | **2** | **3** | **4** |
| Sucrose | 7.0 | - | - | - | - | - |
| HFCS 77 °Brix | - | 9.09 | 9.09 | 6.49 | 3.90 | 3.90 |
| Hesperetin Dihydrochalcone | - | - | 0.0002 | 0.001 | 0.003 | 0.0005 |
| Rebaudioside A | - | - | - | - | - | 0.009 |
| Citric acid anhydrous | 0.137 | 0.137 | 0.137 | 0.137 | 0.137 | 0.137 |
| Orange flavor emulsion | 0.1 | 0.1 | 0.1 | 0.1 | 0.1 | 0.1 |
| Carbonated water | Ad 100 | | | | | |
| *Sugar content [° Brix]* | 7 | 7 | 7 | 5 | 3 | 3 |

| **Evaluation of taste impression** | | | | | | |
|---|---|---|---|---|---|---|
| *Acidic* | 3.5 | 4.5 | 4 | 3.75 | 3.75 | 3.5 |
| *Impact sweetness* | 4 | 2.75 | 3.25 | 3.75 | 3.75 | 4 |
| *Orange* | 6.5 | 5.5 | 6 | 5.75 | 4.5 | 5 |
| *Sweetness intensity* | 6.25 | 4.75 | 5.25 | 6.5 | 7 | 6.75 |
| *Body* | 5 | 3.5 | 4 | 4 | 4 | 4.5 |
| *Juicy* | 5 | 4 | 4.75 | 3.75 | 3.25 | 4 |
| *Lingering* | 1 | 1 | 1 | 4.5 | 5.75 | 4.5 |
| *Peely* | 2.5 | 4 | 3.5 | 3.5 | 3.5 | 3.5 |

**Table 2**

| Carbonated low-caloric soft drinks (all amounts in wt.-percent) | | | | | | |
|---|---|---|---|---|---|---|
| **Ingredient** | **C3** | **C4** | **5** | **6** | **7** | **8** |
| Sucrose | 7.0 | | | | | |
| HFCS 77 °Brix | | 9.09 | 9.09 | 6.49 | 3.90 | 3.90 |
| Hesperetin Dihydrochalcone | - | - | 0.0002 | 0.001 | 0.003 | 0.0005 |
| Rebaudioside A | - | - | - | - | - | 0.009 |
| Cola emulsion | 0.262 | 0.262 | 0.262 | 0.262 | 0.262 | 0.262 |
| Carbonated water | Ad 100 | | | | | |
| *Sugar content [° Brix]* | 7 | 7 | 7 | 5 | 3 | 3 |

| **Evaluation of taste impression** | | | | | | |
|---|---|---|---|---|---|---|
| *Acidic* | 4 | 4.5 | 5 | nd | nd | 5 |
| *Impact sweetness* | 6 | 4 | 5 | nd | nd | 4.5 |
| *Cola* | 6.5 | 6.5 | 6 | nd | nd | 5.5 |
| *Sweetness intensity* | 6.5 | 5.5 | 5.5 | nd | nd | 5.75 |
| *Body* | 5.5 | 5 | 5 | nd | nd | 44 |
| *Spicy* | 4 | 4 | 4 | nd | nd | 34 |
| *Lingering* | 1.5 | 1 | 1 | nd | nd | 3 |

The results clearly indicate that the compositions comprising hesperetin dihydrochalcone in combination with High Fructose Corn Syrup exhibit comparable sweetness and taste impressions as a composition based on sucrose at lower brix values, which means with lower caloric intake.

### FORMULATION EXAMPLES

### FORMULATION EXAMPLE 1

### Spray-Dried Preparation as a Semi-Finished Product for Flavoring of Finished Products (all amounts in wt.-percent)

| **Preparation** | **A** | **B** | **C** | **D** | **E** |
|---|---|---|---|---|---|
| Drinking water | Ad 100 | | | | |
| Maltodextrin from wheat | 31.5 | 29.7 | 28.8 | 27.0 | 27.9 |
| Gum Arabic | 6.1 | 6.1 | 6.1 | 6.1 | 6.1 |
| Hesperetin dihydrochalcone (I) | 1.0 | 0.6 | 0.5 | 0.3 | 0.4 |
| Hesperetin | - | 2.2 | - | - | 1.1 |
| Homoeriodictyol-sodium salt | - | - | - | 5.5 | 3.3 |
| Phloretin | - | - | 3.3 | - | - |

The drinking water is placed in a container and maltodextrin and gum arabic is dissolved in it. Then the flavoring is emulsified in the carrier solution with a Turrax. The temperature of the spray solution should not exceed 30 °C. The mixture is then spray-dried (inlet nominal temperature: 185-195 °C, outlet nominal temperature: 70-75 °C).

### FORMULATION EXAMPLE 2

### Combination with Sweeteners

90 g HFCS and 10 g tagatose are added to 0.5 g of a spray-dried semi-finished product from Formulation example 1 (according to preparation A) and mixed. The product can for example be used as a sweetener with a bitter masking effect for coffee or tea.

### FORMULATION EXAMPLE 3

### Sugar-reduced tomato ketchup (all amounts in wt.-percent)

| **Ingredient** | **Prepar ation** | | | | | | | |
|---|---|---|---|---|---|---|---|---|
| | **A** | **B** | **C** | **D** | **E** | **F** | **G** | **H** |
| Common salt | 2.0 | 2.0 | 2.0 | 2.0 | 2.0 | 2.0 | 2.0 | 2.0 |
| Starch, Farinex WM 55 | 1.0 | 1.0 | 1.0 | 1.0 | 1.0. | 1.0 | 1.0 | 1.0 |
| HFCS | 12.0 | 9.6 | 9.2 | 8.4 | 9.6 | 9.6 | 8.4 | 8.4 |
| Tomato concentrate x 2 | 40.0 | 40.0 | 40.0 | 40.0 | 30.0 | 30.0 | 30.0 | 30.0 |
| Glucose syrup 80 Brix | 18.0 | 18.0 | 18.0 | 18.0 | 18.0 | 18.0 | 18.0 | 18.0 |
| Spirit vinegar 10% | 7.0 | 7.0 | 7.0 | 7.0 | 3.0 | 3.0 | 3.0 | 3.0 |
| Hesperetin dihydrochalcone (I) 0.25% in 1,2-propylene glycol | 0.1 | 0.1 | 0.2 | 0.1 | 0.1 | 0.2 | 0.1 | 0.1 |
| Hesperetin 2,5% in 1,2-Propylene glycol | - | - | - | - | 0.1 | - | 0.2 | - |
| Phloretin 2,5% in 1,2-Propylene glycol | - | - | - | 0.2 | 0.2 | - | - | 0.2 |
| Water | ad 100 | | | | | | | |

The ingredients are mixed in the stated sequence and the finished ketchup is homogenized using an agitator, poured into bottles and sterilized.

### FORMULATION EXAMPLE 4

### Reduced-sugar fruit gums (all amounts in wt.-percent)

| **Ingredient** | **A** | **B** |
|---|---|---|
| Water | Ad 100 | Ad 100 |
| Sucrose | 34.50 | 8.20 |
| HFCS | 31.89 | 30.09 |
| Iso Syrup C* Tru Sweet 01750 (Cerestar GmbH) | 1.50 | 2.10 |
| Gelatin 240 Bloom | 8.20 | 9.40 |
| Polydextrose (Litesse® Ultra, Danisco Cultor GmbH) | - | 24.40 |
| Yellow and red colorings | 0.01 | 0.01 |
| Citric acid | 0.20 | 0.10 |
| Cherry flavoring, containing 0.5 % by weight of hesperetin dihydrochalcone (I) | 0.15 | 0.10 |

| | | |
|---|---|---|
| **[00119]** Note: Polydextrose is itself a non-sweet-tasting polysaccharide with a low calorific value. | | |

### FORMULATION EXAMPLE 5

### Gelatine fruit gum 30% sugar reduced (all amounts in wt. percent)

| **Ingredient** | **A Standard Gum Fruit** | **B Sugar reduced fruit gum** |
|---|---|---|
| Water | Ad 100 | Ad 100 |
| Sucrose | 34.50 | 15.6 |
| Glucose Syrup DE40 | 37.70 | 26.90 |
| Inulin Powder | - | 19.53 |
| HFCS | - | 6.30 |
| Gelatin 240 Bloom | 6.90 | 7.40 |
| Lemon Flavour, containing 0.5 % by weight of hesperetin dihydrochalcone (I) | 0.20 | 0.20 |
| Citric Acid | 1.25 | 1.25 |
| Yellow Colour | 0.05 | 0.05 |
| Rebaudioside A | - | 0.01 |

| | | |
|---|---|---|
| For A: 1. Mix the ingredients 1-3 and cook them up to 118°C 2. Add to the cooked base ingredients 6-9 3. Deposit the mass into starch moulds and let them settle for 24h 4. The Fruit Gums can be oiled/waxed For B: 1. Mix the ingredients 1-5 and cook them up to 118°C 2. Add to the cooked base ingredients 6-10 3. Deposit the mass into starch moulds and let them settle for 24h 4. The Fruit Gums can be oiled/waxed | | |

### FORMULATION EXAMPLE 6

### Gelatine capsules for direct consumption (all amounts in wt.-percent)

| **Ingredient** | **A** | **B** | **C** |
|---|---|---|---|
| **Gelatine shell:** | | | |
| Glycerin | 2.014 | 2.014 | 2.014 |
| Gelatine 240 Bloom | 7.91 | 7.91 | 7.91 |
| Sucralose | 0.065 | 0.065 | 0.065 |
| Allura red | 0.006 | 0.006 | 0.006 |
| Brillant blue | 0.005 | 0.005 | 0.005 |

| **Core composition:** | | | |
|---|---|---|---|
| Vegetable oil-triglyceride (coconut oil fraction) | Ad 100 | Ad 100 | Ad 100 |
| Orange flavoring containing 0.025 % by weight hesperetin dihydrochalcone (I) | 10.00 | 20.00 | 10.00 |
| Rebaudioside A 98 % | 0.05 | 0.05 | - |
| 2-hydroxypropylmenthylcarbonate | 0.33 | 0.20 | - |
| 2-hydroxyethylmenthylcarbonate | - | 0.20 | 1.00 |
| (1R,3R,4S) menthyl-3-carboxylic-acid-N- ethylamide (WS-3) | - | 0.55 | 0.50 |
| (-)-Menthone glycerin acetal (Frescolat MGA) | - | 0.30 | 0.80 |
| Vanillin | 0.07 | - | 0.10 |

The gelatine capsules suitable for direct consumption in HFCS containing foodstuff were prepared according to WO 2004/050069 and had a diameter of 5 mm and the weight ratio of core material to shell material was 90:10. The capsules opened in the mouth in less than 10 seconds and dissolved completely in less than 50 seconds.

### FORMULATION EXAMPLE 7

### Carbonated low-caloric drinks (Flavour Direction: Cola) (all amounts in wt.-percent)

| **Ingredient** | **A** | **B** | **C** | **D** | **E** |
|---|---|---|---|---|---|
| Phosphoric acid 85% | 0.0635 | 0.0635 | 0.0635 | 0.0635 | 0.0635 |
| Citric acid, anhydrous | 0.064 | 0.064 | 0.064 | 0.064 | 0.064 |
| Caffeine | 0.01 | 0.01 | 0.01 | 0.01 | 0.01 |
| Sucrose | 5.0 | - | - | - | 7.0 |
| HFCS 77 °Brix | - | 6.5 | - | 3.9 | - |
| Sucralose | - | 0.0126 | - | - | - |
| Erythritol | - | - | 6.0 | - | - |
| Aspartame | - | - | 0.035 | - | 0.07 |
| Stevioside | - | - | - | 0.0300 | - |
| Acesulfame K | - | - | - | - | 0.07 |
| Sugar coloring | 0.02 | 0.02 | 0.02 | 0.02 | 0.02 |
| Cola type drink emulsion | 0.1445 | 0.1445 | 0.1445 | 0.1445 | 0.1445 |
| Sodium benzoate | 0.0106 | 0.0106 | 0.0106 | 0.0106 | 0.0106 |
| Hesperetin dihydrochalcone (I) 5.0 % in 1,2-propylene glycol | 0.04 | 0.05 | 0.05 | 0.05 | 0.05 |
| Carbonated water | ad 100 | | | | |

In addition, the sugar content of 10 °Brix can be reduced to 7 °Brix while maintaining the sweetness intensity by additionally incorporating rebaudioside A into a combination with HFCS. The combination of hesperetine dihydrochalcone with HFCS is better than the use of HFCS alone (figure 1/2). The preferred sample contains a combination of HFCS + Rebaudioside A + hesperetine dihydrochalcone. It is sugar-reduced and reduced in calories and, at the same time, shows improved sugar-like taste profile as opposed to a sugar-reduced variant with HFCS and Rebaudioside A taken alone (figure 2/2).

The effects found in the above application examples can, if necessary, be modified by all the products of the respective product group, i.e. in particular with respect to chewing gums, candies, gelatin capsules, chewing sweets and tea in bags. The compound according to the invention used in the products of the application examples must also be regarded as a placeholder for the other compounds according to the invention. In addition, the product-specific further constituents in the particular application example are easily interchangeable with other product-type constituents, or can be supplemented by such products. A variety of such product-specific ingredients are disclosed in the above description.

## Claims

1. An aroma composition comprising
(a) hesperetin dihydrochalcone (I) (3-(3-Hydroxy-4-methoxy-phenyl)-1-(2,4,6-trihydroxyphenyl)propan-1-one) 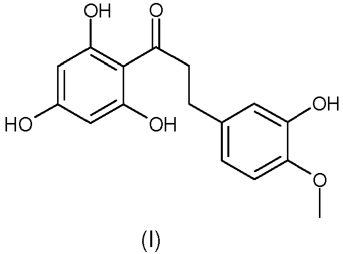 or a salt thereof, and
(b) high fructose corn syrup (HFCS).

2. The composition of Claim 1, further comprising
(c) a sweetener or sweetness enhancer selected from the group consisting of steviosides, fructose, glucose, saccharin, sugar alcohols, cyclamate, neohesperetin dihydrochalcone, erythritol, phloretin or a mixture thereof.

3. The composition of Claim 2, wherein said sweetener is a stevioside.

4. The composition of Claim 3, wherein said stevioside is rebaudioside A.

5. The composition of Claim 1, comprising components (a) and (b) in a weight ratio of from about 0.500:99.500 to about 0.0001:99.9999.

6. The composition of Claim 2, comprising components (a+b) and (c) in a weight ratio of from about 99.99950:0.00050 to about 25:75.

7. The composition of Claim 1 exhibiting sugar content in the range of from 0° Brix to 13° Brix.

8. An oral composition comprising the aroma composition of Claim 1.

9. The composition of Claim 8 being a beverage

10. The composition of Claim 9 being a soft drink.

11. The composition of Claim 9 being a protein shake.

12. The composition of Claim 8 comprising said aroma composition in an amount of from 0.1 to about 30 wt.-percent.

13. A method for providing an oral composition, preferably a beverage with high sweetness and reduced calories, encompassing the following steps:
(i) providing a base for said oral composition or beverage respectively;
(ii) providing the aroma composition of Claim 1;
(iii) blending both compounds; and optionally
(iv) adding carbonic acid to the formulation.

14. The use of the composition of Claim 1 for sweetening an oral composition.

15. The use of the composition of Claim 1 as a substituent for caloric sweeteners.

## Patentansprüche

1. Aroma-Zusammensetzung umfassend
(a) Hesperetindihydrochalkon (I) (3-(3-Hydroxy-4-methoxy-phenyl)-1-(2,4,6-trihydroxyphenyl)propan-1-on) 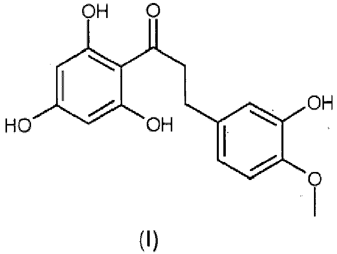 oder ein Salz davon, und
(b) Maissirup mit hohem Fruktosegehalt (HFCS).

2. Zusammensetzung nach Anspruch 1, weiterhin umfassend
(c) einen Süßstoff oder Süßkraftverstärker, ausgewählt aus der Gruppe bestehend aus Steviosiden, Fruktose, Glukose, Saccharin, Zuckeralkoholen, Cyclamat, Neohesperetin-Dihydrochalkon, Erythritol, Phloretin oder eine Mischung davon.

3. Zusammensetzung nach Anspruch 2, wobei der Süßstoff ein Steviosid ist.

4. Zusammensetzung nach Anspruch 3, wobei genanntes Steviosid Rebaudiosid A ist.

5. Zusammensetzung nach Anspruch 1, umfassend die Komponenten (a) und (b) in einem Gewichtsverhältnis von etwa 0,500 : 99,500 bis etwa 0,0001 : 99,9999.

6. Zusammensetzung nach Anspruch 2, umfassend die Komponenten (a+b) und (c) in einem Gewichtsverhältnis von etwa 99,99950 : 0,00050 bis etwa 25 : 75.

7. Zusammensetzung nach Anspruch 1, welche einen Zuckergehalt im Bereich von 0°Brix bis 13°Brix aufweist.

8. Orale Zusammensetzung umfassend die Aroma-Zusammensetzung nach Anspruch 1.

9. Zusammensetzung nach Anspruch 8, welche ein Getränk ist.

10. Zusammensetzung nach Anspruch 9, welche ein Softdrink ist.

11. Zusammensetzung nach Anspruch 9, welche ein Protein-Shake ist.

12. Zusammensetzung nach Anspruch 8 umfassend genannte Aroma-Zusammensetzung in einer Menge von 0,1 bis etwa 30 Gew.-%.

13. Verfahren zur Bereitstellung einer oralen Zusammensetzung, vorzugsweise ein Getränk mit hoher Süße und reduzierten Kalorien, umfassend die folgenden Schritte:
(i) Bereitstellen einer Basis für genannte orale Zusammensetzung oder Getränk;
(ii) Bereitstellen der Aroma-Zusammensetzung aus Anspruch 1;
(iii) Vermischen beider Verbindungen; und optional
(iv) Hinzufügen von Kohlensäure zu der Formulierung.

14. Verwendung der Zusammensetzung nach Anspruch 1 zum Süßen einer oralen Zubereitung.

15. Verwendung der Zusammensetzung nach Anspruch 1 als Ersatz für einen kalorischen Süßstoff.

## Revendications

1. Composition d'arôme comprenant
(a) de l'hespérétine dihydrochalcone (I) (3-(3-hydroxy-4-méthoxy-phényl)-1-(2,4,6-trihydroxyphényl)propan-1-one) 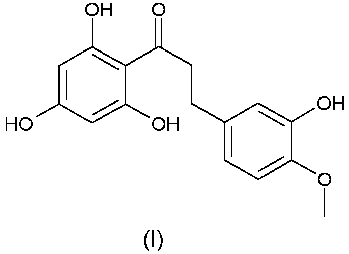 ou un sel de celle-ci, et
(b) du sirop de maïs à haute teneur en fructose (HFCS).

2. Composition selon la revendication 1, comprenant en outre
(c) un édulcorant ou exhausteur de goût sucré choisi dans le groupe constitué par les stéviosides, le fructose, le glucose, la saccharine, les alcools de sucre, le cyclamate, la néo-hespérétine dihydrochalcone, l'érythritol, la phlorétine ou un mélange de ceux-ci.

3. Composition selon la revendication 2, dans laquelle ledit édulcorant est un stévioside.

4. Composition selon la revendication 3, dans laquelle ledit stévioside est le rébaudio-side A.

5. Composition selon la revendication 1, comprenant les composants (a) et (b) dans un rapport pondéral de 0,500 : 99,500 à peu près à 0,0001 : 99,9999 à peu prés.

6. Composition selon la revendication 2, comprenant les composants (a+b) et (c) dans un rapport pondéral de 99,99950 : 0,00050 à peu près à 25 : 75 à peu près.

7. Composition selon la revendication 1, présentant une teneur en sucre se situant dans la gamme allant de 0° Brix à 13° Brix.

8. Composition orale comprenant la composition d'arôme selon la revendication 1.

9. Composition selon la revendication 8, qui est une boisson.

10. Composition selon la revendication 9, qui est une boisson sans alcool.

11. Composition selon la revendication 9, qui est un shake protéiné.

12. Composition selon la revendication 8, comprenant ladite composition d'arôme en une quantité comprise entre 0,1 et environ 30 pour cent en poids.

13. Procédé destiné à fournir une composition orale, de préférence une boisson à haute douceur et à teneur réduite en calories, comprenant les étapes suivantes consistant à:
(i) fournir une base pour ladite composition orale ou bien la boisson;
(ii) fournir la composition d'arôme selon la revendication 1 ;
(iii) mélanger les deux composés; et, en option,
(iv) ajouter de l'acide carbonique à la formulation.

14. Utilisation de la composition selon la revendication 1 pour édulcorer une composition orale.

15. Utilisation de la composition selon la revendication 1 en tant que substituant d'édulcorants caloriques.
